# EUROPÄISCHE PATENTANMELDUNG

(11) **EP 1 653 214 A1**
(43) Veröffentlichungstag der Anmeldung: **03.05.2006**
(21) Anmeldenummer: 05109773.1
(22) Anmeldetag: 20.10.2005
(51) Int. Cl.: G01N 15/02, G01N 21/53, G01N 33/24

(54) **Verfahren zur Bestimmung von Eigenschaften einer Fasersuspension**

(30) Priorität: 26.10.2004 DE 102004051960
(71) Anmelder: Voith Paper Patent GmbH, 89522 Heidenheim (DE)
(72) Erfinder: Hardt, Niels, 89522 Heidenheim (DE); Schwarz, Michael, 89522 Heidenheim (DE)

(57) **Zusammenfassung**

Die Erfindung betrifft ein Verfahren und einen Sensor (1) zur Bestimmung von Messwerten von Eigenschaften einer Suspension (2) in einer Maschine zur Herstellung einer Faserstoffbahn bei dem Licht mit der Suspension wechselwirkt, wobei der Sensor ein Streulichtsignal (14) und ein Durchlichtsignal (15) aufnimmt und Messwerte (18,19) zumindest einer Eigenschaft durch Auswertung des Streulichtsignals (14) und des Durchlichtsignals (15) bestimmt werden.

## Beschreibung

Diese Erfindung betrifft ein Verfahren zur Bestimmung von Messwerten von Eigenschaften einer Suspension in einer Maschine zur Herstellung und / oder Veredelung einer Faserstoffbahn, insbesondere Papier, nach dem Oberbegriff des Patentanspruchs 1 sowie einen Sensor und eine Maschine zur Herstellung und / oder Veredelung einer Faserstoffbahn.

Aus dem Stand der Technik sind im Wesentlichen zwei optische Verfahren zur Messung von Eigenschaften, insbesondere der Stoffdichte und des Aschegehalts, einer Suspension in einer Maschine zur Herstellung und / oder Veredelung einer Faserstoffbahn bekannt.

Beim ersten Verfahren werden Eigenschaften der Suspension mit Durchlicht, beim zweiten Verfahren mit Streulicht gemessen.

Bei der Durchlichtmessung wird in der Regel monochromatisch gemessen. Der Sender und der Empfänger sind ca. 1,5 bis 3mm voneinander entfernt, sodass die dazwischen befindliche Suspension gemessen werden kann. Beim Durchlichtverfahren werden salop gesagt "Schatten" von Partikeln in der Suspension gemessen. Die Durchlichtmessung ist unabhängig von der Partikelart. Aufgrund eines kleinen Messfensters, der Lichtstrahl hat einen Durchmesser zwischen 0,2mm und 0,5mm, ist das Durchlichtmessverfahren stark abhängig vom Flockungsgrad und von der Strömung der Suspension, wodurch Messfehler entstehen können.

Bei der Streulichtmessung wird das von Partikeln in der Suspension zurück gestreute Licht eines großen, aber undefinierten Volumenbereichs gemessen. Die Streulichtmessung ist stark abhängig von der Partikelart und deren Streuintensität.

Es ist die Aufgabe der vorliegenden Erfindung ein Verfahren vorzuschlagen, mit dem Eigenschaften einer Suspension mit höherer Genauigkeit über einen weiteren Messbereich als bei den bekannten Verfahren ermittelt werden können. Des weiteren besteht die Aufgabe der vorliegenden Erfindung darin, einen entsprechenden Sensor vorzuschlagen.

Die Aufgabe wird durch ein Verfahren zur Bestimmung von Messwerten von Eigenschaften einer Suspension in einer Maschine zur Herstellung einer Faserstoffbahn mittels eines Sensors bei dem Licht mit der Fasersuspension wechselwirkt dadurch gelöst, dass der Sensor ein Streulichtsignal und ein Durchlichtsignal aufnimmt und dass Messwerte zumindest einer Eigenschaft durch Auswertung des Streulichtsignals und des Durchlichtsignals bestimmt werden.

Der Erfindung liegt die Idee zugrunde, Messwerte der zumindest einen Eigenschaft dadurch genauer zu bestimmen, indem Signale unterschiedlicher Messprinzipien, der Streulichtmessung und der Durchlichtmessung, zusammengeführt werden, die bzgl. der gleichen Eigenschaft der Suspension unterschiedlichen Informationsgehalt haben. Dadurch wird der Informationsgehalt auf Basis der Signalwerte zur Bestimmung der Messwerte der zumindest einen Eigenschaft erhöht, wodurch diese genauer bestimmt werden können. Hierbei werden auch "falsche" Signale des einen Messprinzips durch "richtige" Signale des anderen Messprinzips bei der Bestimmung von Messwerten der zumindest einen Eigenschaft der Suspension erkannt und eliminiert.

Hierdurch ist es möglich, die Vorteile beider Messprinzipien zu vereinen und Nachteile zu eliminieren.

Vorteilhafte Ausgestaltungen und Weiterbildungen der Erfindung sind in den Unteransprüchen angegeben.

Für eine optimale Steuerung bei der Herstellung von Faserstoffbahnen, insbesondere Papier, ist es wichtig, mehrere Eigenschaften der Suspensionen in einer Papiermaschine zu kennen. So sind bspw. die Stoffdichte und der Aschegehalt besonders wichtig. Demzufolge sieht eine bevorzugte Ausführungsform vor, dass Messwerte von zwei Eigenschaften, insbesondere die Stoffdichte und der Aschegehalt, durch Auswertung des Streulichtsignals und des Durchlichtsignals bestimmt werden.

Mit dem erfindungsgemäßen Verfahren können aber auch Messwerte anderer Eigenschaften einer Suspension, wie bspw. Feinstoffanteil oder dgl. bestimmt werden.

Insbesondere zur Verfahrens- und Prozesssteuerung bei der Herstellung der Faserstoffbahn ist es sinnvoll, wenn Messwerte einer Eigenschaft der Faserstoffbahn im Betrieb gemessen werden, d.h. wenn die Suspension zur Messung der zumindest einen Eigenschaft nicht aus dem Produktionskreislauf entnommen werden muss. Demzufolge sieht eine besonders bevorzugte Ausführungsform der Erfindung vor, dass die Messung Online durchgeführt wird, insbesondere in einer die Suspension führenden Rohrleitung, die an den Produktionskreislauf angeschlossen ist.

Um die statistische Auswertbarkeit der gemessenen Signale zu erhöhen, sieht eine weitere Ausgestaltung der Erfindung vor, dass das Streusignal und das Durchlichtsignal hoch aufgelöst, insbesondere mit einer Samplingrate von mehr als 2kHz, vorzugsweise von mehr als 4 kHz, aufgenommen werden.

Durch das erfindungsgemäße Verfahren werden die Messwerte der Stoffdichte vorzugsweise in einem Bereich von 0,01% bis 4,0% mit einer Messgenauigkeit von 0,005% bis 0,01 % bestimmt.

Des weiteren werden durch das erfindungsgemäße Verfahren die Messwerte des Aschegehalts in einem Bereich von 0,01 % bis 1,0% mit einer Messgenauigkeit von 0,005% bis 0,01% bestimmt.

Um die Messgenauigkeit und den Messbereich zu erhöhen, sieht eine bevorzugte Ausgestaltung der Erfindung vor, dass das Streulichtsignal und das Durchlichtsignal bei mehreren und / oder unterschiedlichen Wellenlängen gemessen werden. Dies kann bedeuten, dass das Streulichtsignal und / oder das Durchlichtsignal bei einer diskreten Wellenlänge oder mehreren diskreten Wellenlängen oder in einem Wellenlängenbereich gemessen werden. Vorzugsweise ist deshalb vorgesehen, dass das Streulichtsignal und bei Wellenlängen aus dem Bereich von 250nm bis 1000nm und das Durchlichtsignal bei Wellenlängen aus dem Bereich von 600nm bis 1000nm gemessen werden.

Vorzugsweise wird der Sensor unter Verwendung von Labormesswerten der zumindest einen Eigenschaft kalibriert. Auf diese Weise lassen sich Abweichungen der vom Sensor gemessenen Messdaten umgehend und einfach, bspw. automatisch korrigieren.

Sollen zwei Eigenschaften der Suspension bestimmt werden, ist es sinnvoll, wenn der Sensor unter Verwendung von Labormesswerten von zwei Eigenschaften, insbesondere der Stoffdichte und des Aschegehalts, kalibriert wird.

In einer vorteilhaften Weiterbildung des Verfahrens werden zur Kalibrierung weitere Prozessinformationen und / oder weitere Messwerte verwendet. Dies bedeutet, dass die vom Sensor bestimmten Messwerte der zumindest einen Eigenschaft anhand von anderen Sensoren ermittelten Werten gewichtet und korrigiert werden können. Dadurch lassen sich Abhängigkeiten einer von einem Sensor gemessenen Größe von anderen Prozessinformationen und / oder Messwerten berücksichtigen. Hierbei lässt sich auch bspw. ein zeitlicher Drift von Messwerten dergestalt berücksichtigen, dass unmittelbar vor den aktuellen Messwerten gemessene Werte stärker in die Korrektur eingehen als ältere Messwerte.

Besonders bevorzugt werden Messwerte der zumindest einen Eigenschaft im Stoffauflauf und / oder im von der Siebpartie weggeführten Wasser und / oder im von der Pressenpartie weggeführten Wasser bestimmt. Auf Basis dieser Messwerte kann der Herstellungsprozess optimal gesteuert werden.

Um auch Produktionsschwankungen in Maschinenquerrichtung erfassen zu können, sieht eine bevorzugte Ausführungsform der Erfindung vor, dass Messwerte der zumindest einen Eigenschaft sektional über die Maschinenbreite ermittelt werden.

Als besonders geeignet erweist sich, wenn die Kalibrierung mit den folgenden multivariaten Regressionsverfahren erfolgt: Partial Least Square Regression (PLSR) oder Principle Component Regression (PCR). Diese Verfahren sind allgemein bekannt. Zum Nachweis wird hier beispielhaft auf das Buch "Multivariate Calibration" von H.Martens, T.Naes, Verlag John Wiley & Sohn, Chichester, New York, Brisbane, Toronto, Singapore, 1998 verwiesen. Darin wird bspw. die Principle Component Regression (PCR) beschreiben.

Vorteilhafterweise erfolgt die Kalibrierung des Sensors in einem vorgegebenen Zeitabstand. Hierdurch wird erreicht, dass Änderungen der Messwerte im Laufe der Zeit in einem bestimmten Rahmen gehalten werden.

Die Erfindung betrifft des weiteren einen Sensor zur Bestimmung von Messwerten von Eigenschaften einer Suspension in einer Maschine zur Herstellung einer Faserstoffbahn bei dem Licht mit der Suspension in Wechselwirkung tritt, der dadurch gekennzeichnet ist, dass der Sensor eine Streulichtmesseinheit und eine Durchlichtmesseinheit aufweist, deren Signale einer gemeinsamen Auswerteeinheit zuführbar sind.

Hierdurch wird ein Sensor geschaffen, der ein Streulichtsignal und ein Durchlichtsignal aufnimmt und der Messwerte zumindest einer Eigenschaft durch Auswertung des Streulichtsignals und des Durchlichtsignals ermittelt.

Um den Sensor möglichst kompakt und bzgl. Umgebungsbedingungen unempfindlich ausbilden zu können ist es vorteilhaft, wenn die Streulichtmesseinheit und die Durchlichtmesseinheit in einem Gehäuse zusammengefasst sind.

Die Erfindung betrifft des weiteren eine Maschine zur Herstellung und / oder Veredelung einer Faserstoffbahn mit einem erfindungsgemäßen Sensor.

Um On-Line Messungen durchführen zu können, ist der Sensor in einer eine Suspension führenden Rohrleitung, insbesondere einer Durchflussleitung angeordnet.

Die Erfindung wird anhand der folgenden schematischen Zeichnungen näher erläutert. Es zeigen:
- Fig. 1: einen erfindungsgemäßen Sensor mit dem das erfindungsgemäße Verfahren durchführbar ist,
- Fig. 2: eine Papiermaschine mit erfindungsgemäßen Sensoren.

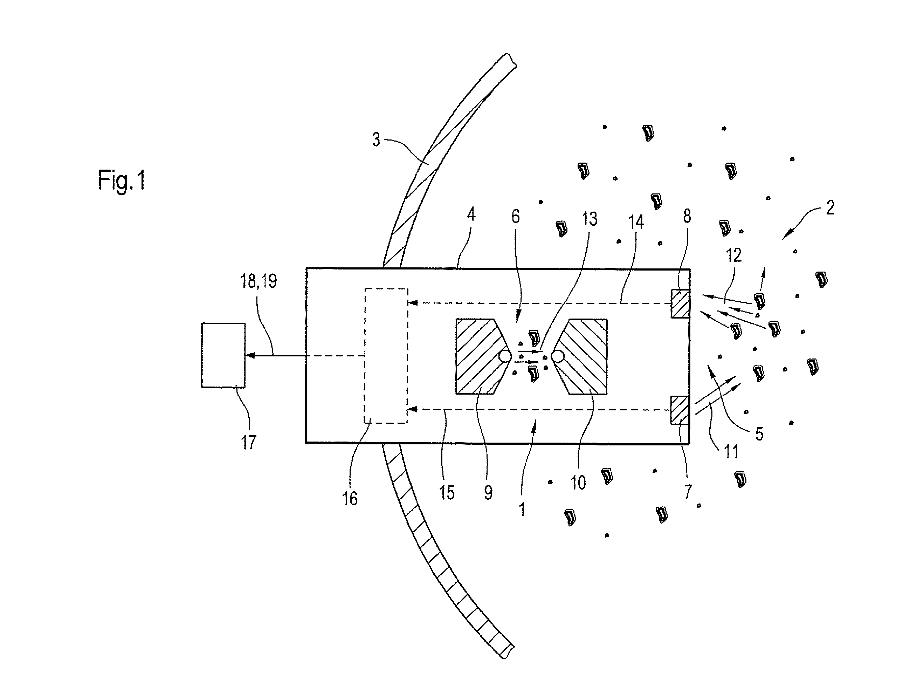

Die Figur 1 zeigt einen erfindungsgemäßen Sensor 1, welcher in einer eine Suspension 2 führenden Rohrleitung 3 eingebaut ist in Seitenansicht.

Die Rohrleitung 3 ist nur abschnittweise dargestellt. Wie zu erkennen ist, weist der Sensor 1 seitlich in die Rohrleitung 3, die von der Suspension 2 durchströmt wird (Die Strömungsrichtung ist senkrecht zur Zeichnungsebene).

Der Sensor weist ein Sensorgehäuse 4 auf. Die im Bereich des Sensorgehäuses 4 außen liegenden Komponenten sind in der Darstellung der Figur 1 schraffiert dargestellt. Die sich innerhalb des Sensorgehäuses 4 befindlichen Komponenten, sind strichliert dargestellt.

Der Sensor weist eine Streulichtmesseinheit 5 und eine Durchlichtmesseinheit 6 auf.

Die Streulichtmesseinheit 5 und die Durchlichtmesseinheit 6 sind beim erfindungsgemäßen Sensor 1 in einem Gehäuse, nämlich dem Sensorgehäuse 4 zusammengefasst.

Die Streulichteinheit 5 weist einen Sender 7 und einem Empfänger 8 auf. Der Sender 7 sendet Licht 11 bei einer diskreten Wellenlänge oder bei mehreren diskreten Wellenlängen oder in einem oder mehreren Wellenlängenbereich(en) in den sich vor ihm erstreckenden Halbraum aus. Die diskreten Wellenlängen bzw. der oder die Wellenlängenbereich entstammen hierbei aus einem Wellenlängenbereich von 250nm bis 1000nm. Das Licht 11 wechselwirkt mit der Suspension 2 und das bei der Wechselwirkung entstehende Streulicht 12, welches in Richtung des Empfängers 8 gerichtet ist, wird von dem Empfänger 8 als Streulichtsignal 14 detektiert.

Die Durchlichteinheit 6 weist einen Sender 9 und einen Empfänger 10 auf. Der Sender 9 sendet Licht 13 gerichtet in Richtung des Empfängers 10, wobei die Lichtintensität durch Wechselwirkung mit der Suspension 2 abgeschwächt und vom Empfängers 10 als Durchlichtsignal 15 detektiert wird. Das Licht wird bei einer diskreten Wellenlänge oder bei mehreren diskreten Wellenlängen oder in einem oder mehreren Wellenlängenbereich(en) ausgesendet. Die diskrete(n) Wellenlänge(n) bzw. der oder die Wellenlängenbereich(e) entstammen hierbei aus einem Wellenlängenbereich von 600nm bis 1000nm.

Zur Bestimmung von Messwerten 18 der Stoffdichte und Messwerten 19 des Aschegehalts der Suspension 2 werden das vom Detektor 8 aufgenommene Streulichtsignal 14 und das vom Detektor 10 aufgenommene Durchlichtsignal 15 einer Auswerteeinheit 16 zugeführt. In der Auswerteeinheit 16 werden dann Messwerte 18 der Stoffdichte und Messwerte 19 des Aschegehalts der Suspension 2 durch Auswertung des Streulichtsignals 14 und des Durchlichtsignals 15 bestimmt.

Aufgrund der Anordnung des Sensors in der mit der Suspension 2 durchströmten Rohrleitung 3 und aufgrund der Tatsache, dass der Sensor derart ausgelegt ist, dass dieser zumindest eine Messung pro Sekunde durchführen kann, können die Messungen On-Line durchgeführt werden.

Der Sensor 1 ist derart ausgelegt, dass das Streusignal 14 und das Durchlichtsignal 15 hoch aufgelöst aufgenommen werden kann, insbesondere mit einer Samplingrate von mehr als 2kHz, vorzugsweise von mehr als 4 kHz. Somit können statistische Ausreißer heraus gefiltert werden.

Mit dem erfindungsgemäßen Sensor 1 können Messwerte 18 der Stoffdichte in einem Bereich von 0,01% bis 4,0% mit einer Messgenauigkeit von 0,005% bis 0,01% bestimmt werden. Ferner können mit dem erfindungsgemäßen Sensor 1 Messwerte 19 des Aschegehalts in einem Bereich von 0,01% bis 1,0% mit einer Messgenauigkeit von 0,005% bis 0,01% bestimmt werden.

Des weiteren wird der Sensor 1 unter Verwendung von Labormesswerten der Stoffdichte und des Aschegehalts der Suspension 2 kalibriert. Auf diese Weise lassen sich Abweichungen der vom Sensor 1 gemessenen Messdaten 18, 19 umgehend und einfach, bspw. automatisch korrigieren. Die Kalibrierung wird hierbei mittels einer multivariaten Regression, bspw. mittels der Partial Least Square Regression (PLSR) durchgeführt.

Zur Kalibrierung des Sensors 1 werden des weiteren weitere Prozessinformationen und / oder weitere Messwerte, wie bspw. Eigenschaften der produzierten Faserstoffbahn oder dgl. verwendet.

Die Messwerte 18, 19 werden einer Steuerungs- und / oder Regelungseinheit 17 einer Papiermaschine zugeführt.

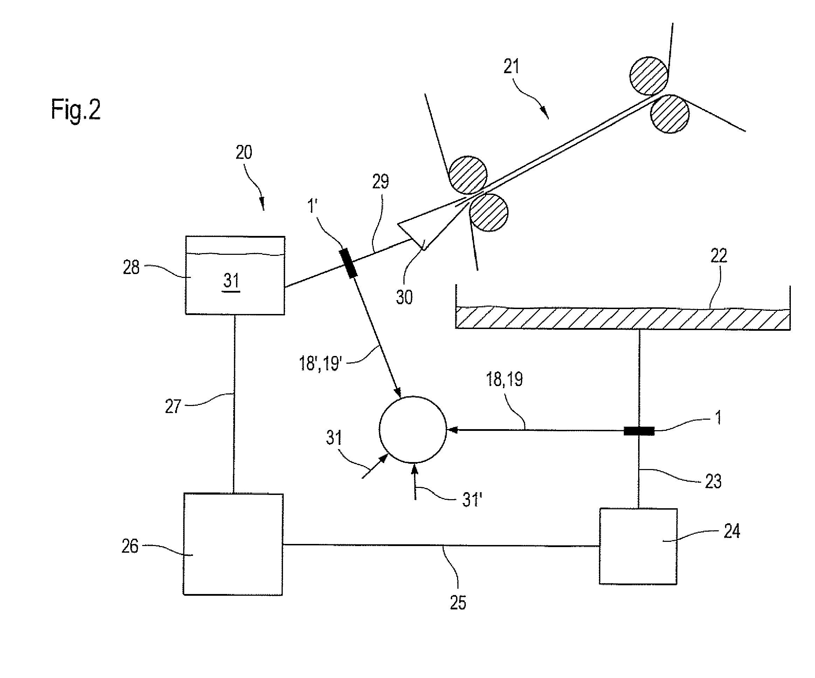

Die Fig. 2 zeigt einen Abschnitt einer Papiermaschine 20 mit zwei erfindungsgemäßen Sensoren 1 und 1'.

Das aus der Siebpartie 21 gelangende Siebwasser 22 wird über eine Rohrleitung 23 einem Siebwasserauffangbehälter 24 zugeführt. In der Rohrleitung 23 ist einer der Sensoren 1 angeordnet mit dem On-Line Messdaten 18, 19 der Stoffdichte und des Aschegehalts im Siebwasser 22 gemessen werden.

Das Siebwasser 22 wird vom Siebwasserauffangbehälter 24 über eine Rohrleitung 25 zur Stoffaufbereitung 26 geführt. Die in der Stoffaufbereitung 26 hergestellte Fasersuspension 31 kommt über eine Rohrleitung 27 in die Maschinenbütte 28 und wird von dort über eine Rohrleitung 29 einem Stoffauflauf 30 in der Siebpartie 21 der Papiermaschine 20 zugeführt.

In der Rohrleitung 29 ist der andere Sensor 1' angeordnet, mit dem On-Line Messdaten 18', 19' der Stoffdichte und des Aschegehalts im Siebwasser 22 gemessen werden.

Die von den beiden Sensoren 1 und 1' gemessenen Messdaten 18, 19 und 18', 19' werden der Steuer- und / oder Auswerteeinheit 17 der Papiermaschine 20 zugeführt, welche die Papiermaschine 20 auf Basis der Messwerte 18, 19, 18'und 19' und auf Basis weiterer Daten 31, 31' steuert und regelt.

## Patentansprüche

1. Verfahren zur Bestimmung von Messwerten von Eigenschaften einer Suspension in einer Maschine zur Herstellung einer Faserstoffbahn mittels eines Sensors bei dem Licht mit der Suspension wechselwirkt,
**dadurch gekennzeichnet,**
**dass** der Sensor ein Streulichtsignal und ein Durchlichtsignal aufnimmt und dass Messwerte zumindest einer Eigenschaft durch Auswertung des Streulichtsignals und des Durchlichtsignals bestimmt werden.

2. Verfahren nach Anspruch 1,
**dadurch gekennzeichnet,**
**dass** Messwerte von zwei Eigenschaften, insbesondere die Stoffdichte und der Aschegehalt, durch Auswertung des Streulichtsignals und des Durchlichtsignals bestimmt werden.

3. Verfahren nach Anspruch 1 oder 2,
**dadurch gekennzeichnet,**
**dass** die Messung Online durchgeführt wird.

4. Verfahren nach einem der vorangegangenen Ansprüche,
**dadurch gekennzeichnet,**
**dass** das Streusignal und das Durchlichtsignal hoch aufgelöst, insbesondere mit einer Samplingrate von mehr als 2kHz, vorzugsweise von mehr als 4 kHz, aufgenommen wird.

5. Verfahren nach einem der vorangegangenen Ansprüche,
**dadurch gekennzeichnet,**
**dass** Messwerte der Stoffdichte in einem Bereich von 0,01% bis 4,0% mit einer Messgenauigkeit von 0,005% bis 0,01% bestimmt werden.

6. Verfahren nach einem der vorangegangenen Ansprüche,
**dadurch gekennzeichnet,**
**dass** Messwerte des Aschegehalts in einem Bereich von 0,01 % bis 1,0% mit einer Messgenauigkeit von 0,005% bis 0,01 % bestimmt werden.

7. Verfahren nach einem der vorangegangenen Ansprüche,
**dadurch gekennzeichnet,**
**dass** das Streulichtsignal und bei Wellenlängen aus dem Bereich von 250nm bis 1000nm und das Durchlichtsignal bei Wellenlängen aus dem Bereich von 600nm bis 1000nm gemessen werden.

8. Verfahren nach einem der vorangegangenen Ansprüche,
**dadurch gekennzeichnet,**
**dass** der Sensor unter Verwendung von Labormesswerten der zumindest einen Eigenschaft kalibriert wird.

9. Verfahren nach Anspruch 8,
**dadurch gekennzeichnet,**
**dass** der Sensor unter Verwendung von Labormesswerten von zwei Eigenschaften, insbesondere der Stoffdichte und des Aschegehalts, kalibriert wird.

10. Verfahren nach Anspruch 8 oder 9,
**dadurch gekennzeichnet,**
**dass** zur Kalibrierung weitere Prozessinformationen und / oder weitere Messwerte verwendet werden.

11. Verfahren nach einem der vorangegangenen Ansprüche,
**dadurch gekennzeichnet,**
**dass** Messwerte der zumindest einen Eigenschaft im Stoffauflauf und / oder im von der Siebpartie weggeführten Wasser und / oder im von der Pressenpartie weggeführten Wasser bestimmt werden.

12. Verfahren nach einem der vorangegangenen Ansprüche,
**dadurch gekennzeichnet,**
**dass** Messwerte der zumindest einen Eigenschaft sektional über die Maschinenbreite ermittelt werden.

13. Verfahren nach einem der Ansprüche 8 bis 12,
**dadurch gekennzeichnet,**
**dass** die Kalibrierung mittels einer multivariaten Regression, insbesondere mittels Partial Least Square Regression (PLSR) oder Principle Component Regression (PCR), erfolgt.

14. Verfahren nach einem der Ansprüche 8 bis 13,
**dadurch gekennzeichnet,**
**dass** die Kalibrierung des Sensors in einem vorgegebenen Zeitabstand erfolgt.

15. Sensor zur Bestimmung von Messwerten von Eigenschaften einer Suspension in einer Maschine zur Herstellung einer Faserstoffbahn bei dem Licht mit der Suspension wechselwirkt,
**dadurch gekennzeichnet,**
**dass** der Sensor eine Streulichtmesseinheit und eine Durchlichtmesseinheit aufweist, deren Signale einer gemeinsamen Auswerteeinheit zuführbar sind.

16. Sensor nach Anspruch 15,
**dadurch gekennzeichnet,**
**dass**, die Streulichtmesseinheit und die Durchlichtmesseinheit in einem Gehäuse zusammengefasst sind.

17. Maschine zur Herstellung und / oder Veredelung einer Faserstoffbahn mit einem Sensor nach einem der Ansprüche 14 bis 17.

18. Maschine nach Anspruch 17,
**dadurch gekennzeichnet,**
**dass** der Sensor in einer eine Suspension führenden Rohrleitung, insbesondere einer Durchflussleitung angeordnet ist.
